(19) Europäisches Patentamt
European Patent Office
Office européen des brevets

(11) EP 1 560 807 B1

(12) **EUROPÄISCHE PATENTSCHRIFT**

(45) Veröffentlichungstag und Bekanntmachung des Hinweises auf die Patenterteilung:
**10.02.2010 Patentblatt 2010/06**

(21) Anmeldenummer: **03810964.1**

(22) Anmeldetag: **05.11.2003**

(51) Int Cl.:
***C07C 217/52*** (2006.01) ***C07C 213/06*** (2006.01)
***C07C 221/00*** (2006.01)

(86) Internationale Anmeldenummer:
**PCT/EP2003/012312**

(87) Internationale Veröffentlichungsnummer:
**WO 2004/043902 (27.05.2004 Gazette 2004/22)**

(54) **4-HYDROXYMETHYL-1-ARYL-CYCLOHEXYLAMIN-DERIVATIVE**

4-HYDROXYMETHYL-1-ARYL-CYCLOHEXYLAMINE DERIVATIVES

DERIVES DE 4-HYDROXYMETHYL-1-ARYL-CYCLOHEXYLAMINE

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**LT LV**

(30) Priorität: **12.11.2002 DE 10252872**
**14.11.2002 DE 10253323**

(43) Veröffentlichungstag der Anmeldung:
**10.08.2005 Patentblatt 2005/32**

(73) Patentinhaber: **Grünenthal GmbH**
**52078 Aachen (DE)**

(72) Erfinder:
- **HINZE, Claudia**
**53175 Bonn (DE)**
- **Die andere Erfinder haben auf ihre Nennung verzichtet.**

(56) Entgegenhaltungen:
**WO-A-99/36421 US-A- 4 115 589**

- **PAQUETTE, LEO A. ET AL.: TETRAHEDRON LETTERS, Bd. 34, Nr. 22, 1993, Seiten 3523-3526, XP002273512**

Bemerkungen:
Die Akte enthält technische Angaben, die nach dem Eingang der Anmeldung eingereicht wurden und die nicht in dieser Patentschrift enthalten sind.

EP 1 560 807 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft 4-Hydroxymethyl-1-Aryl-Cyclohexylamin-Derivate, Verfahren zu deren Herstellung, Arzneimittel enthaltend diese Verbindungen und die Verwendung von 4-Hydroxymethyl-1-Aryl-Cyclohexylamin-Derivaten zur Herstellung von Arzneimitteln.

Das Heptadekapeptid Nociceptin ist ein endogener Ligand des ORL1 (Opioid-Receptor-Like)-Rezeptors (Meunier et al., Nature 377, 1995, S. 532-535), der zu der Familie der Opioid Rezeptoren gehört und in vielen Regionen des Gehirns und des Rückenmarks zu finden ist und eine hohe Affinität für den ORL1-Rezeptor aufweist. Der ORL1-Rezeptor ist homolog zu den μ, κ und δ Opioid-Rezeptoren und die Aminosäuresequenz des Nociceptin-Peptids weist eine starke Ähnlichkeit mit denen der bekannten Opioidpeptide auf. Die durch das Nociceptin induzierte Aktivierung des Rezeptors führt über die Kopplung mit $G_{i/o}$-Proteinen zu einer Inhibierung der Adenylatcyclase (Meunier et al., Nature 377, 1995, S. 532-535).

Das Nociceptin-Peptid zeigt nach intercerebroventicularer Applikation eine pronociceptive und hyperalgetische Aktivität in verschiedenen Tiermodellen (Reinscheid et al., Science 270, 1995, S. 792-794). Diese Befunde können als Hemmung der stressinduzierten Analgesie erklärt werden (Mogil et al., Neuroscience 75, 1996, S. 333-337). In diesem Zusammenhang konnte auch eine anxiolytische Aktivität des Nociceptin nachgewiesen werden (Jenck et al., Proc. Natl. Acad. Sci. USA 94, 1997, 14854-14858).

Auf der anderen Seite konnte in verschiedenen Tiermodellen, insbesondere nach intrathekaler Applikation, auch ein antinociceptiver Effekt von Nociceptin gezeigt werden. Nociceptin wirkt antinociceptiv in verschiedenen Schmerzmodellen, beispielsweise im Tail Flick-Test in der Maus (King et al., Neurosci. Lett., 223, 1997, 113-116. In Modellen für neuropathische Schmerzen konnte ebenfalls eine antinociceptive Wirkung von Nociceptin nachgewiesen, die insofern besonders interessant ist, als dass die Wirksamkeit von Nociceptin nach Axotomie von Spinalnerven zunimmt. Dies steht im Gegensatz zu den klassischen Opioiden, deren Wirksamkeit unter diesen Bedingungen abnimmt (Abdulla und Smith, J. Neurosci., 18, 1998, S. 9685-9694).

Der ORL1-Rezeptor ist außerdem noch an der Regulation weiterer physiologischer und pathophysiologischer Prozesse beteiligt. Hierzu gehören unter anderem Lernen und Gedächtnisbildung (Manabe et al., Nature, 394, 1997, S. 577-581), Hörvermögen (Nishi et al., EMBO J., 16, 1997, S. 1858-1864) sowie zahlreiche weitere Prozesse. In einem Übersichtsartikel von Calo et al. (Br.J. Pharmacol., 129, 2000, 1261 - 1283) wird ein Überblick über die Indikationen oder biologischen Vorgänge gegeben, in denen der ORL1-Rezeptor eine Rolle spielt oder mit hoher Wahrscheinlichkeit spielen könnte. Genannt werden u.a.: Analgesie, Stimulation und Regulation der Nahrungsaufnahme, Einfluß auf μ-Agonisten wie Morphin, Behandlung von Entzugserscheinungen, Reduzierung des Suchtpotentials von Opioiden, Anxiolyse, Modulation der Bewegungsaktivität, Gedächtnis-Störungen, Epilepsie; Modulation der Neurotransmitter-Ausschüttung, insbesondere von Glutamat, Serotonin und Dopamin, und damit neurodegenerative Erkrankungen; Beeinflußung des cardiovaskulären Systems, Auslösung einer Erektion, Diurese, Antinatriurese, Elektrolyt-Haushalt, arterieller Blutdruck, Wasserspeicher-Krankheiten, intestinale Motilität (Diarrhoe), relaxierende Effekte auf die Atemwege, Mikturations Reflex (Harninkontinenz). Weiter wird die Verwendung von Agonisten und Antagonisten als Anoretika, Analgetika (auch in Coadministration mit Opioiden) oder Nootropika diskutiert.

Entsprechend vielfältig sind die Anwendungsmöglichkeiten von Verbindungen, die an den ORL1-Rezeptor binden und diesen aktivieren oder inhibieren. Neben diesem spielen gerade im Bereich der Schmerztherapie, aber auch bei anderen der genannten Indikationen, Opioidrezeptoren wie der μ-Rezeptor und andere Subtypen eine große Rolle. Entsprechend ist es günstig, wenn die Verbindung auch Wirkung an diesen Opioidrezeptoren zeigen.

Die Anmeldung WO 99/36421 offenbart 4-(2-Keto-1-benzimidazolinyl)-piperidin-Derivate, für die eine ORL1-agonistische Wirkung beschrieben wird.

Aufgabe der vorliegenden Erfindung war es, Arzneimittel zur Verfügung zu stellen, die auf das Nociceptin/ORL1-Rezeptor-System wirken und damit für Arzneimittel insbesondere zur Behandlung der verschiedenen mit diesem System nach dem Stand der Technik in Verbindung stehenden Krankeiten bzw. zum Einsatz in den dort genannten Indikationen geeignet sind.

Gegenstand der Erfindung sind daher 4-Hydroxymethyl-1-Aryl-Cyclohexylamin-Derivate der allgemeinen Formel I,

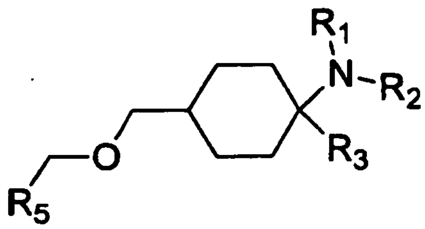

, worin

$R^1$ und $R^2$ unabhängig voneinander für H; $C_{1-8}$-Alkyl gesättigt oder ungesättigt, verzweigt oder unverzweigt, einfach oder mehrfach substituiert oder unsubstituiert, stehen,

oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,

wobei $R^6$ H; $C_{1-8}$-Alkyl gesättigt oder ungesättigt, verzweigt oder unverzweigt, einfach oder mehrfach substituiert oder oder unsubstituiert bedeutet;

$R^3$ für $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenen Aryl, $C_{3-8}$-Cycloalkyl oder Heteroaryl, jeweils unsubstituiert oder einfach oder mehrfach substituiert, steht;

$R^5$ für $C_{3-8}$-Cycloalkyl, Aryl oder Heteroaryl, jeweils unsubstituiert oder einfach oder mehrfach substituiert; $-CH_2R^{12}$, $-CH_2-CH_2R^{12}$, $-CH_2-CH_2-CH_2R^{12}$, steht

wobei $R^{12}$ $C_{3-8}$-Cycloalkyl, Aryl oder Heteroaryl, jeweils unsubstituiert oder einfach oder mehrfach substituiert, bedeutet,

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate.

Alle diese erfindungsgemäßen Verbindungen zeigen eine gute Bindung an den ORL1-Rezeptor, aber auch an andere Opiatrezeptoren.

Im Sinne dieser Erfindung versteht man unter Alkyl- bzw. Cykloalkyl-Resten gesättigte und ungesättigte (aber nicht aromatische), verzweigte, unverzweigte und cyclische Kohlenwasserstoffe, die unsubstituiert oder ein- oder mehrfach substituiert sein können. Dabei steht $C_{1-2}$-Alkyl für C1- oder C2-Alkyl, $C_{1-3}$-Alkyl für C1-, C2- oder C3-Alkyl, $C_{1-4}$-Alkyl für C1-, C2-, C3- oder C4-Alkyl, $C_{1-5}$-Alkyl für C1-, C2-, C3-, C4- oder C5-Alkyl, $C_{1-6}$-Alkyl für C1-, C2-, C3-, C4-, C5- oder C6-Alkyl, $C_{1-7}$Alkyl für C1-, C2-, C3-, C4-, C5-, C6- oder C7-Alkyl, $C_{1-8}$-Alkyl für C1-, C2-, C3-, C4-, C5-, C6-, C7- oder C8-Alkyl, $C_{1-10}$-Alkyl für C1-, C2-, C3-, C4-, C5-, C6-, C7-, C8,- C9- oder C10-Alkyl und $C_{1-18}$-Alkyl für C1-, C2-, C3-, C4-, C5-, C6-, C7-, C8,- C9-, C10-, C11-, C12-, C13-, C14-, C15-, C16-, C17- oder C18-Alkyl. Weiter steht $C_{3-4}$-Cycloalkyl für C3- oder C4-Cycloalkyl, $C_{3-5}$-Cycloalkyl für C3-, C4- oder C5-Cycloalkyl, $C_{3-6}$-Cycloalkyl für C3-, C4-, C5- oder C6-Cycloalkyl, $C_{3-7}$-Cycloalkyl für C3-, C4-, C5-, C6- oder C7-Cycloalkyl, $C_{3-8}$-Cycloalkyl für C3-, C4-, C5-, C6-, C7- oder C8-Cycloalkyl, $C_{4-5}$-Cycloalkyl für C4- oder C5-Cycloalkyl, $C_{4-6}$-Cycloalkyl für C4-, C5- oder C6-Cycloalkyl, $C_{4-7}$-Cycloalkyl für C4-, C5-, C6- oder C7-Cycloalkyl, $C_{5-6}$-Cycloalkyl für C5- oder C6-Cycloalkyl und $C_{5-7}$-Cycloalkyl für C5-, C6- oder C7-Cycloalkyl. In Bezug auf Cycloalkyl umfaßt der Begriff auch gesättigte Cycloalkyle, in denen ein oder 2 Kohlenstoffatome durch ein Heteroatom, S, N oder O ersetzt sind. Unter den Begriff Cycloalkyl fallen aber insbesondere auch ein- oder mehrfach, vorzugsweise einfach, ungesättigte Cycloalkyle ohne Heteroatom im Ring, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch

Adamantyl, $CHF_2$, $CF_3$ oder $CH_2OH$ sowie Pyrazolinon, Oxopyrazolinon, [1.4]Dioxan oder Dioxolan.

Dabei versteht man im Zusammenhang mit Alkyl und Cycloalkyl - solange dies nicht ausdrücklich anders definiert ist - unter dem Begriff substituiert im Sinne dieser Erfindung die Substitution mindestens eines (gegebenenfalls auch mehrerer) Wasserstoffreste(s) durch F, Cl, Br, I, $NH_2$, SH oder OH, wobei unter "mehrfach substituiert" bzw. "substituiert" bei mehrfacher Substitution zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von $CF_3$ oder an verschiedenen Stellen wie im Falle von -CH(OH)-CH=CH-$CHCl_2$. Besonders bevorzugte Substituenten sind hier F, Cl und OH. In Bezug auf Cycloalkyl kann der Wasserstoffrest auch durch $OC_{1-3}$-Alkyl oder $C_{1-3}$-Alkyl (jeweils ein- oder mehrfach substituiert oder unsubstituiert), insbesondere Methyl, Ethyl, n-Propyl, i-Propyl, $CF_3$, Methoxy oder Ethoxy, ersetzt sein.

Unter dem Begriff $(CH_2)_{3-6}$ ist $-CH_2-CH_2-CH_2-$, $-CH_2-CH_2-CH_2-CH_2-$, $-CH_2-CH_2-CH_2-CH_2-CH_2-$ und $CH_2-CH_2-CH_2-CH_2-CH_2-CH_2-$ zu verstehen, unter $(CH_2)_{1-4}$ ist $-CH_2-$, - $CH_2CH_2-$, $-CH_2-CH_2-CH_2-$ und $-CH_2-CH_2-CH_2-CH_2-$ zu verstehen, unter $(CH_2)_{4-5}$ ist - $CH_2-CH_2-CH_2-CH_2-$ und $-CH_2-CH_2-CH_2-CH_2-CH_2-$ zu verstehen, etc.

Unter einem Aryl-Rest werden Ringsysteme mit mindestens einem aromatischen Ring aber ohne Heteroatome in auch nur einem der Ringe verstanden. Beispiele sind Phenyl-, Naphthyl-, Fluoranthenyl-, Fluorenyl-, Tetralinyl- oder Indanyl, insbesondere 9H-Fluorenyl- oder Anthracenyl-Reste, die unsubstituiert oder einfach oder mehrfach substituiert sein können.

Unter einem Heteroaryl-Rest werden heterocyclische Ringsysteme mit mindestens einem ungesättigten Ring verstanden, die ein oder mehrere Heteroatome aus der Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo[1,2,5] thiadiazol, Benzothiazol, Indol, Benzotriazol, Benzodioxolan, Benzodioxan, Carbazol, Indol und Chinazolin aufgeführt.

Dabei versteht man im Zusammenhang mit Aryl und Heteroaryl unter substituiert die Substitution des Aryls oder Heteroaryls mit $R^{22}$, $OR^{22}$ einem Halogen, vorzugsweise F und/oder Cl, einem $CF_3$, einem CN, einem $NO_2$, einem $NR^{23}R^{24}$, einem $C_{1-6}$-Alkyl (gesättigt), einem $C_{1-6}$-Alkoxy, einem $C_{3-8}$-Cycloalkoxy, einem $C_{3-8}$-Cydoalkyl oder einem $C_{2-6}$-Alkylen.

Dabei steht der Rest $R^{22}$ für H, einen $C_{1-10}$-Alkyl-, vorzugsweise einen $C_{1-6}$-Alkyl-, einen Aryl- oder Heteroaryl- oder für einen über $C_{1-3}$-Alkyl, gesättigt oder ungesättigt, oder einen über eine $C_{1-3}$-Alkyl-Gruppe gebundenen Aryl- oder Heteroaryl-Rest, wobei diese Aryl und Heteroarylreste nicht selbst mit Aryl- oder Heteroaryl-Resten substituiert sein dürfen,

die Reste $R^{23}$ und $R^{24}$, gleich oder verschieden, für H, einen $C_{1-10}$-Alkyl-, vorzugsweise einen $C_{1-6}$-Alkyl-, einen Aryl-, einen Heteroaryl- oder einen über $C_{1-3}$-Alkyl, gesättigt oder ungesättigt, gebundenen Aryl- oder Heteroaryl-Rest bedeuten, wobei diese Aryl und Heteroarylreste nicht selbst mit Aryl- oder Heteroaryl-Resten substituiert sein dürfen,

oder die Reste $R^{23}$ und $R^{24}$ bedeuten zusammen $CH_2CH_2OCH_2CH_2$, $CH_2CH_2NR^{25}CH_2CH_2$ oder $(CH_2)_{3-6}$, und

der Rest $R^{25}$ für H, einen $C_{1-10}$-Alkyl-, vorzugsweise einen $C_{1-6}$-Alkyl-, einen Aryl-, oder Heteroaryl- Rest oder für einen über $C_{1-3}$-Alkyl, gesättigt oder ungesättigt, gebundenen Aryl- oder Heteroaryl-Rest, wobei diese Aryl und Heteroarylreste nicht selbst mit Aryl- oder Heteroaryl-Resten substituiert sein dürfen.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/ oder Säugetier-veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, 1,1-Dioxo-1,2-dihydro1b6-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, a-Liponsäure, Acetylglycin, Acetylsalicylsäure, Hippursäure und/oder Asparaginsäure. Besonders bevorzugt ist das Hydrochlorid-Salz und das Citrat-Salz.

Unter dem Begriff des mit einer physiologisch verträglichen Säure gebildeten Salzes versteht man im Sinne

dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid und das Citrat. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1$\lambda^6$-benzo[*d*]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, $\alpha$-Liponsäure, Acetylglycin, Acetylsalicylsäure, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit $NH_4^+$, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder Säugetier - veträglich ist. Besonders bevorzugt sind die Salze der Alkali- und Erdalkalimetalle aber auch $NH_4^+$, insbesondere aber (Mono-) oder (Di-) Natrium-, (Mono-) oder (Di-) Kalium-, Magnesium- oder Calzium-Salze.

Erfindüngsgemäß sind 4-Hydroxymethyl-1-Aryl-Cyclohexylamin-Derivate, worin

$R^1$ und $R^2$ unabhängig voneinander für H; $C_{1-8}$-Alkyl, gesättigt oder ungesättigt, verzweigt oder unverzweigt, einfach oder mehrfach substituiert oder unsubstituiert, stehen;

oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,

wobei $R^6$ H; $C_{1-8}$-Alkyl, gesättigt oder ungesättigt, verzweigt oder unverzweigt, einfach oder mehrfach substituiert oder unsubstituiert, bedeutet,

vorzugsweise

$R^1$ und $R^2$ unabhängig voneinander für H; $C_{1-4}$-Alkyl, gesättigt oder ungesättigt, verzweigt oder unverzweigt, einfach oder mehrfach substituiert oder unsubstituiert, stehen; wobei $R^1$ und $R^2$ nicht beide H sein dürfen,

oder die Reste $R^1$ und $R^2$ zusammen einen Ring bilden und $(CH_2)_{4-5}$ bedeuten.

Besonders bevorzugt sind 4-Hydroxymethyl-1-Aryl-Cyclohexylamin-Derivate,
worin

$R^1$ und $R^2$ unabhängig voneinander für Methyl oder Ethyl stehen oder die Reste $R^1$ und $R^2$ zusammen einen Ring bilden und $(CH_2)_5$ bedeuten C3094-PCT.doc oder $R^1$ und $R^2$ unabhängig voneinander für $CH_3$ oder H stehen, wobei $R^1$ und $R^2$ nicht gleichzeitig H bedeuten.

Bevorzugt im Sinne dieser Erfindung sind auch 4-Hydroxymethyl-1-Aryl-Cyclohexylamin-Derivate, worin

$R^3$ für $C_{3-8}$-Cycloalkyl, Aryl oder Heteroaryl, jeweils unsubstituiert oder einfach oder mehrfach substituiert; über eine gesättigte oder ungesättigte, unverzweigte, substituierte oder unsubstituierte $C_{1-2}$-Alkyl-Gruppe gebundenen Aryl, $C_{3-8}$-Cycloalkyl oder Heteroaryl, jeweils unsubstituiert oder einfach oder mehrfach substituiert, steht;

vorzugsweise

$R^3$ Cyclopentyl, Cyclohexyl, Phenyl, Benzyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyridyl, Pyrimidyl oder Pyrazinyl, jeweils unsubstituiert oder einfach oder mehrfach substituiert; über eine gesättigte, unverzweigte $C_{1-2}$-Alkyl-Gruppe gebundenen $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert, bedeutet;

insbesondere

$R^3$ Phenyl, Furyl, Thiophenyl, Naphthyl, Benzyl, Benzofuranyl, Indolyl, Indanyl, Benzodioxanyl, Benzodioxolanyl, Pyridyl, Pyrimidyl, Pyrazinyl oder Benzothiophenyl, jeweils unsubstituiert oder einfach oder mehrfach substituiert; über eine gesättigte, unverzweigte $C_{1-2}$-Alkyl-Gruppe gebundenen Phenyl, Furyl oder Thiophenyl, jeweils unsubstituiert oder einfach oder mehrfach substituiert, bedeutet.

Besonders bevorzugt sind 4-Hydroxymethyl-1-Aryl-Cyclohexylamin-Derivate, worin $R^3$ für Phenyl, Thiophenyl, Pyridyl oder Benzyl, jeweils substituiert oder unsubstituiert, steht, ganz besonders bevorzugt für Phenyl.
Weiterhin bevorzugt sind 4-Hydroxymethyl-1-Aryl-Cyclohexylamin-Derivate, worin

$R^5$ für $C_{3-8}$-Cycloalkyl, Aryl oder Heteroaryl, jeweils unsubstituiert oder einfach oder mehrfach substituiert, steht;

vorzugsweise

$R^5$ Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert, bedeutet;

insbesondere

$R^5$ Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert, bedeutet.

Ganz besonders bevorzugt ist $R^5$ ausgewählt ist aus Phenyl oder Indolyl, jeweils unsubstituiert, ein- oder mehrfach substituiert, wobei unsubstiuiertes Phenyl oder Indolyl oder einfach mit Methyl, Methoxy, Chlor, Fluor oder $CF_3$ in para-Stellung substituiertes Phenyl oder mit mit Methyl, Methoxy, Chlor, Fluor oder $CF_3$ in 5-Position substiuiertes Indolyl erfindungsgemäß insbesondere bevorzugt sind.
Außerdem bevorzugt sind 4-Hydroxymethyl-1-Aryl-Cyclohexylamin-Derivate, worin

$R^5$ für $-CH_2R^{12}$, $-CH_2-CH_2R^{12}$, $-CH_2-CH_2-CH_2R^{12}$ steht

vorzugsweise

$R^5$ $-CH_2R^{12}$, $-CH_2-CH_2R^{12}$ bedeutet

insbesondere

$R^5$ $-CH_2R^{12}$ bedeutet,

wobei

$R^{12}$ für $C_{3-8}$-Cycloalkyl, Aryl oder Heteroaryl, jeweils unsubstituiert oder einfach oder mehrfach substituiert, steht;

vorzugsweise

$R^{12}$ Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert, bedeutet;

insbesondere

$R^{12}$ für Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert, bedeutet.

Ganz besonders bevorzugt sind 4-Hydroxymethyl-1-Aryl-Cyclohexylamin-Derivate aus der Gruppe
(4-Benzyloxymethyl-1-phenylcyclohexyl)dimethylamin Hydrochlorid, unpolareres Diastereoisomer
(4-Benzyloxymethyl-1-phenylcyclohexyl)dimethylamin Hydrochlorid, polareres Diastereoisomer
[4-(4-Fluorbenzyloxymethyl)-1-phenylcyclohexyl]dimethylamin Hydrochlorid, unpolareres Diastereoisomer
[4-(4-Fluorbenzyloxymethyl)-1-phenylcyclohexyl]dimethylamin Hydrochlorid, polareres Diastereoisomer,
gegebenenfalls auch in Form ihrer Mischungen.

Die erfindungsgemäßen Substanzen wirken beispielsweise auf den im Zusammenhang mit verschiedenen Erkrankungen relevanten ORL1-Rezeptor, sodass sie sich als pharmazeutischer Wirkstoff in einem Arzneimittel eignen.Ein weiterer Gegenstand der Erfindung sind daher Arzneimittel enthaltend wenigstens ein 4-Hydroxymethyl-1-Aryl-Cyclohexylamin-Derivat.

Die erfindungsgemäßen Arzneimittel enthalten neben mindestens einem erfindungsgemäßen 4-Hydroxymethyl-1-Aryl-Cyclohexylamin-Derivat gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhatative Applikation Lösungen, Suspensionen, leicht rekonstituierbare Trockenzubereitungen sowie Sprays. Erfindungsgemäße 4-Hydroxymethyl-1-Aryl-Cyclohexylamin-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen 4-Hydroxymethyl-1-Aryl-Cyclohexylamin-Derivate verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 1000 mg/kg, bevorzugt 0,05 bis 5 mg/kg wenigstens eines erfindungsgemäßen 4-Hydroxymethyl-1-Aryl-Cyclohexylamin-Derivats appliziert.

Für alle vorstehenden Formen der erfindungsgemäßen Arzneimittel kann es vorteilhaft sein, wenn das Arzneimittel neben wenigstens einem 4-Hydroxymethyl-1-Aryl-Cyclohexylamin-Derivat noch einen weiteren Wirkstoff, insbesondere ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittel liegt ein enthaltenes erfindungsgemäßes 4-Hydroxymethyl-1-Aryl-Cyclohexylamin-Derivat als reines Diastereomer und/oder Enantiomer, als Razemat oder als nicht-äquimolare oder äquimolare Mischung der Diastereomere und/oder Enantiomere vor.

Wie in der Einleitung am Stand der Technik abzulesen, wurde der ORL1-Rezeptor insbesondere im Schmerzgeschehen identifiziert. Entsprechend können erfindungsgemäße 4-Hydroxymethyl-1-Aryl-Cyclohexylamin-Derivate zur Herstellung eines Arzneimittels zur Behandlung von Schmerz, insbesondere von akutem, neuropathischem oder chronischem Schmerz, verwendet werden.

Ein weiterer Gegenstand der Erfindung ist daher die Verwendung eines erfindungsgemäßen 4-Hydroxymethyl-1-Aryl-Cyclohexylamin-Derivats zur Herstellung eines Arzneimittels zur Behandlung von Schmerz, insbesondere von akutem, viszeralem, neuropathischem oder chronischem Schmerz.

Ein weiterer Gegenstand der Erfindung ist daher die Verwendung eines erfindungsgemäßen 4-Hydroxymethyl-1-Aryl-Cyclohexylamin-Derivats zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn ein verwendetes 4-Hydroxymethyl-1-Aryl-Cyclohexylamin-Derivat als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung ist ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wiksamen Dosis eines erfindungsgemäßen 4-Hydroxymethyl-1-Aryl-Cyclohexylamin-Derivats, oder eines erfindungsgemäßen Arzneimittels.

Ein weiterer Gegenstand der Erfindung ist ein Verfahren zur Herstellung der erfindungsgemäßen 4-Hydroxymethyl-1-Aryl-Cyclohexylamin-Derivate wie in der folgenden Beschreibung und Beispielen ausgeführt. Insbesondere geeignet sind dabei die beiden im folgenden als Verfahren I und Verfahren II genannten Verfahren zur Herstellung eines erfindungsgemäßen 4-Hydroxymethyl-1-Aryl-Cyclohexylamin-Derivats ausgehend von geeignet substituierten 4-Aminocyclohexanonen mit folgenden Schritten,

wobei $R^1$, $R^2$, $R^3$ und $R^5$ die für erfindungsgemäße Verbindungen gemäß Formel I angegebene Bedeutung haben,

und

$R^{01}$ und $R^{02}$ unabhängig voneinander für eine Schutzgruppe stehen oder die für erfindungsgemäße Verbindungen gemäß Formel I für $R^1$ und $R^2$ angegebene Bedeutung haben:

Verfahren I:

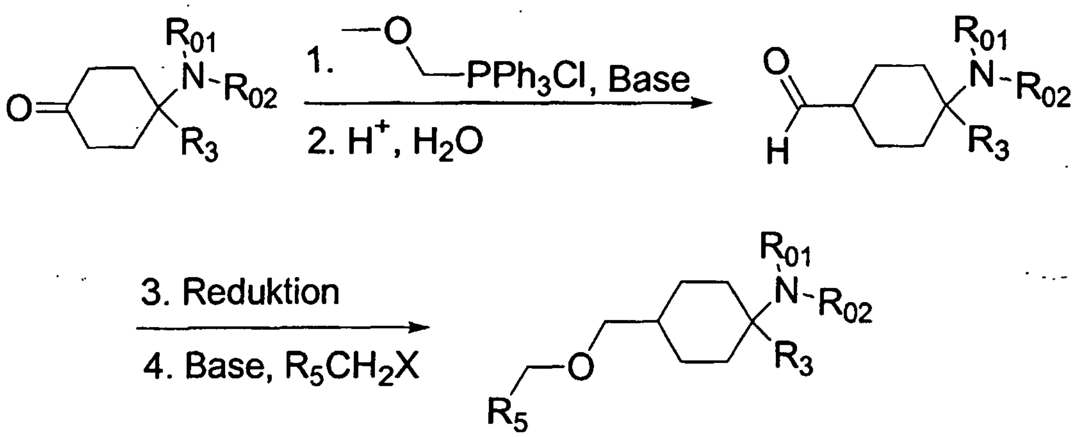


Verfahren II:

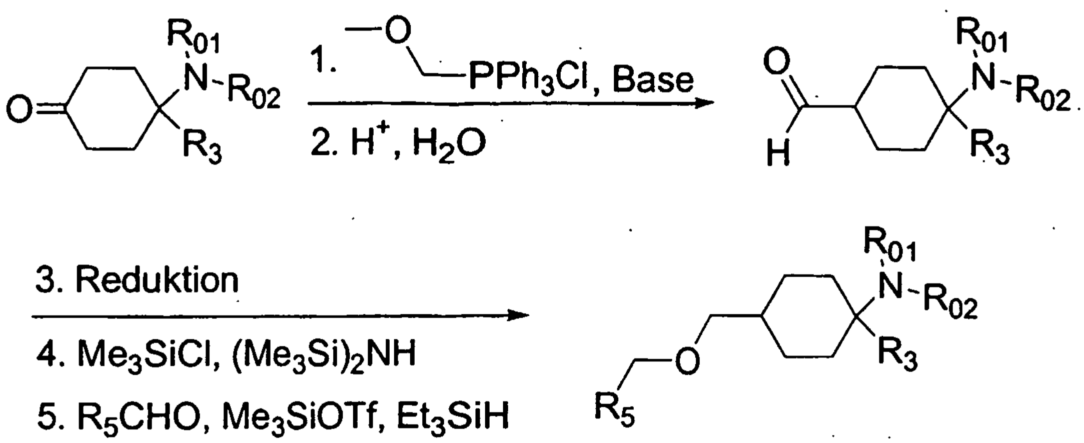


In beiden Verfahren werden 4-Aminocyclohexanon-Derivate mit Methoxytriphosphoniumchlorid und einer Base, beispielsweise Natriumhydrid, und anschließend mit wäßriger Säure, beispielsweise HCl, zu den entsprechenden Aldehyden umgesetzt.

In Verfahren I werden 4-Aminocyclohexancarbaldehyd-Derivate mit einem Reduktionsmittel, beispielsweise einem Hydrid wie Natrium- oder Lithiumborhydrid, Natriumcyanoborhydrid, Natriumtriacetoxyborhydrid, Diiobutylaluminiumhydrid, Lithium-tri-(sec.-butyl)borhydrid (L-Selectride®) oder Lithiumaluminiumhydrid, gegebenenfalls in Anwesenheit von Lewis-Säuren, beispielsweise $ZnCl_2$, $Ni(OAc)_2$ oder $CoCl_2$, oder durch katalytische Hydrierung an Edelmetallen, beispielsweise Palladium oder Platin, mit Wasserstoff reduziert und darauf folgend in Gegenwart von Base, beispielsweise Kalium-tert-butanolat, Lithium- oder Natriumhydrid, Kalium- oder Natriumhydroxid, Butyllithium oder anderen basischen Organometallverbindungen wie Grignardreagenzien, beispielsweise Ethylmagnesiumchlorid oderbromid, mit einem Alkylierungsmittel

$$R^5CH_2X \text{ mit } X = Cl, Br$$

zu den erfindungsgemäßen 4-Hydroxymethyl-1-Aryl-Cyclohexylamin-Derivaten umgesetzt.

In Verfahren II werden 4-Aminocyclohexancarbaldehyd-Derivate durch Zugabe eines Reduktionsmittels, beispielsweise einem Hydrid wie Natrium- oder Lithiumborhydrid, Natriumcyanoborhydrid, Natriumtriacetoxyborhydrid, Diisobutylaluminiumhydrid, Lithium-tri-(sec.-butyl)borhydrid (L-Selectride®) oder Lithiumaluminiumhydrid, gegebenenfalls in Anwesenheit von Lewis-Säuren, beispielsweise $ZnCl_2$, $Ni(OAc)_2$ oder $CoCl_2$, oder durch katalytische Hydrierung an Edelmetallen, beispielsweise Palladium oder Platin, mit Wasserstoff zu den entsprechenden Alkoholen reduziert, und die Alkohole darauf folgend mit Trimethylchlorsilan, Hexamethyldisilazan sowie anschließend einem Aldehyd $R^5CHO$ unter Zugabe von Trimethylsilyltriflat und Triethylsilan zu den erfindungsgemäßen 4-Hydroxymethyl-1-Aryl-Cyclohexylamin-Derivaten umgesetzt.

Die Herstellung geeigneter 4-Aminocyclohexanone ist aus der Literatur bekannt (Lednicer et al., J. Med. Chem., 23, 1980, 424-430; WO 0290317).

Die Isolierung der erfindungsgemäßen Verbindungen durch Säulenchromatographie mit Kieselgel als stationärer Phase und Ethylacetat, Methanol, aus Ethylacetat und Methanol oder Gemischen aus Ethylacetat und Diethylether als Laufmittel führt zu einer Auftrennung der unterschiedlich polaren Diastereoisomeren. Diese wurden aufgrund ihrer Laufzeit bei der Trennung als "unpolarstes Diastereoisomer" (kürzeste Laufzeit) bis "polarstes Diastereoisomer" (längste Laufzeit) charakterisiert.

Im folgenden wird die Erfindung weiter durch Beispiele erläutert, ohne sie darauf zu beschränken.

## Beispiele

Die Ausbeuten der hergestellten Verbindungen sind nicht optimiert.

Alle Temperaturen sind unkorrigiert.

Die Angabe "Ether" bedeutet Diethylether, "EE" Ethylacetat, "DCM" Dichlormethan, "DMF" Dimethylformamid, "DMSO" Dimethylsulfoxid und "THF" Tetrahydrofuran. Die Angabe "Äquivalente" bedeutet Stoffmengenäquivalente, "Smp." Schmelzpunkt bzw. Schmelzbereich, "Zers." Zersetzung, "RT" Raumtemperatur , "abs." absolut (wasserfrei), ,"rac." racemisch, "konz." konzentriert, "min" Minuten, "h" Stunden, "d" Tage, "Vol.%" Volumenprozent, "m%" Massenprozent und "M" ist eine Konzentrationsangabe in mol/l.

Als stationäre Phase für die Säulenchromatographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Laufmitteln für chromatographische Untersuchungen sind stets in Volumen/ Volumen angegeben.

**Beispiel 1:** (4-Benzyloxymethyl-1-phenylcyclohexyl)dimethylamin Hydrochlorid, unpolareres Diastereoisomer

4-Dimethylamino-4-phenylcyclohexancarbaldehyd - Methode A:

Unter Argon wurde Methoxymethyltriphenylphosphoniumchlorid (6,3 g, 18,4 mmol) in DMF (25 ml) gelöst und Natriumhydrid (60 massenprozentig in Mineralöl, 737 mg, 18,4 mmol) zugegeben. 4-Dimethylamino-4-phenylcyclohexanon (2,0 g, 9,2 mmol), gelöst in 25 ml DMF, wurde in 30 min zugetropft und die Suspension 3 d bei RT gerührt. Zur Aufarbeitung wurde die Suspension langsam in eine mit Eiswasser gekühlte 2M HCl (50 ml) gegossen, bei RT 2 h gerührt und anschließend mit Diethylether (5 x 25 ml) und EE (6 x 20 ml) extrahiert. Die wässrige Phase wurde dann mit 1 M NaOH auf pH 10-11 gebracht und mit EE (5 x 20 ml) extrahiert. Die vereinigten Extrakte wurden mit Natriumsulfat getrocknet, filtriert und eingeengt. Der Rückstand war 4-Dimethylamino-4-phenylcyclohexancarbaldehyd (2,0 g braunes Öl). im Diastereoisomerenverhältnis 55 : 45 ($^1$H-NMR).

4-Dimethylamino-4-phenylcyclohexancarbaldehyd - Methode B:

Methoxymethyltriphenylphosphoniumchlorid (2,7 g, 8 mmol) wurde in abs. DMSO (5 ml) und THF (10 ml) unter Argon gelöst und auf < 0 °C abgekühlt. Kalium-tert-butanolat (900 mg, 8 mmol), gelöst in THF (10 ml), wurde zugetropft und 15 min bei 0 °C nachgerührt. Nach Erwärmung auf RT wurde in THF (6 ml) gelöstes 4-Dimethylamino-4-phenylcyclohexanon (870 mg, 4 mmol) zugetropft und über Nacht gerührt. Zur Aufarbeitung wurde das Reaktionsgemisch unter Eiswasserkühlung mit Wasser (5 ml) und 5,5M HCl (15 ml) versetzt und gerührt. Nach 1 h wurde bei RT mit Ether (10 x 20 ml) extrahiert. Die wässrige Phase wurde mit 5M NaOH auf pH 10 gebracht und mit EE (5 x 15 ml) extrahiert. Die vereinigten Extrakte wurden getrocknet, filtriert und eingeengt. Es wurde ein 32 : 68-Gemisch der Diastereoisomeren von 4-Dimethylamino-4-phenylcyclohexancarbaldehyd erhalten (850 mg braunes Öl).

4-Dimethylamino-4-phenylcyclohexancarbaldehyd - Methode C:

In reinem abs. THF wurde analog Methode B bei analoger Ansatzgröße, Durchführung und Ausbeute ein Diastereoisomerenverhältnis von 60 : 40 erzielt.

(4-Dimethylamino-4-phenylcyclohexyl)methanol:

4-Dimethylamino-4-phenylcyclohexancarbaldehyd (2,35 g, 10,2 mmol) wurde in einer Mischung aus 1M NaOH (10,2 ml) und Ethanol/Wasser (2:1, 60 ml) unter Argon Schutzgas 30 min bei RT gerührt. Anschließend wurde tropfenweise eine Lösung von Natriumborhydrid (769 mg, 20,3 mmol) in Wasser (40 ml) bei RT innerhalb 60 min zugegeben und 2 h gerührt. Zur Aufarbeitung wurde Ethanol im Vakuum entfernt und der wässrige Rückstand mit EE (6 x 20 ml) extrahiert. Die vereinigten Extrakte wurden mit gesättigter NaCl-Lösung (2 x 20 ml) gewaschen, getrocknet und eingeengt. Der erhaltene Rückstand (2,2 g braunes Öl) war ein Gemisch der beiden Diastereoisomeren von (4-Dimethylamino-4-phenylcyclohexyl)methanole.

(4-Benzyloxymethyl-1-phenylcyclohexyl)dimethylamin Hydrochlorid, unpolareres Diastereoisomer:

4-Dimethylamino-4-phenylcyclohexyl)methanol (1,8 g, 7,7 mmol) wurde in abs. DMSO (10 ml) vorgelegt und eine Lösung von Kalium-tert-butanolat (1,73 g, 15,4 mmol) in DMSO (20 ml) innerhalb 15 min zugetropft. Es wurde auf 50 °C erwärmt und 30 min gerührt, bevor Benzylchlorid (1,46 g, 11,6 mmol) in DMSO (10 ml) innerhalb 15 min zugetropft wurden. Nach Rühren über Nacht bei 50 °C wurde Wasser (20 ml) zugegeben und mit Ether (3 x 30 ml) gefolgt von DCM (3 x 30 ml) extrahiert. Die vereinigten Extrakte wurden mit Wasser (20 ml) gewaschen, getrocknet, filtriert und eingeengt. Der erhaltene Rückstand (2,85 g) wurde mit EE (30 ml) zum Rückfluß erhitzt, heiß filtriert und das Filtrat über Nacht bei 4 °C aufbewahrt. Der ausgefallene Feststoff wurde abgesaugt und getrocknet (960 mg eines Gemisches der diastereoisomeren Zielprodukte), die Mutterlauge zur Trockne eingeengt und der erhaltene Rückstand (1,37 g) mit Ether unter steigendem Zusatz von Methanol an Kieselgel chromatographiert. Es wurden 308 mg des unpolareren Diastereoisomers von (4-Benzyloxymethyl-1-phenylcyclohexyl)dimethylamin erhalten, die in 2-Butanon (2 ml) gelöst wurden.

Durch Zugabe von Chlortrimethylsilan (129 µl) und Wasser (9 µl) wurde ein öliger Rückstand erhalten, der nach Trocknung 258 mg des korrespondierenden Hydrochlorids ergab.

**Beispiel 2:** (4-Benzyloxymethyl-1-phenylcyclohexyl)dimethylamin Hydrochlorid, polareres Diastereoisomer

Wie für Beispiel 1 beschrieben wurden auch 278 mg des polareren Diastereoisomers von (4-Benzyloxymethyl-1-phenylcyclohexyl)dimethylamin erhalten, die in analoger Weise in 305 mg des korrespondierenden Hydrochlorids überführt wurden.

**Beispiel 3:** [4-(4-Fluorbenzyloxymethyl)-1-phenylcyclohexyl]dimethylamin Hydrochlorid, unpolareres Diastereoisomer

Wie für Beispiel 1 beschrieben wurde auch 4-Fluorbenzylchlorid (1,67 g, 11,6 mmol) mit 4-Dimethylamino-4-phenylcyclohexyl)methanol (1,8 g, 7,7 mmol) und Kalium-tert-butanolat (1,73 g, 15,4 mmol) umgesetzt. Nach chromatographischer Reinigung des Rohprodukts (2,78 g) wurden 480 mg des unpolareren Diastereoisomers von [4-(4-Fluorbenzyloxymethyl)-1-phenylcyclohexyl]dimethylamin erhalten, die in analoger Weise in 348 mg des korrespondierenden Hydrochlorids überführt wurden.

**Beispiel 4:** [4-(4-Fluorbenzyloxymethyl)-1-phenylcyclohexyl]dimethylamin Hydrochlorid, polareres Diastereoisomer

4-Dimethylamino-4-phenylcyclohexyl)methanol (2,2 g, 9,4 mmol) wurde in abs. THF (25 ml) gelöst und bei RT mit Hexamethyldisilazan (10,3 ml, 49,8 mmol) und Chlortrimethylsilan (2,35 ml, 25,1 mmol) versetzt. Nach 18 h bei RT wurde im Vakuum zur Trockne eingedampft. Der Rückstand wurde in Ether (35 ml) aufgenommen und mit 1,1M $NaHCO_3$-Lösung (2 x 5 ml) gewaschen. Die organische Phase wurde mit Ether auf 50 ml verdünnt, über Natriumsulfat getrocknet, filtriert und eingeengt. Das erhaltene Dimethyl-(1-phenyl-4-trimethylsilanyloxymethylcyclohexyl)amin (2,4 g braunes Öl) wurde ohne weitere Aufreinigung verwendet.
Unter Argon wurde 4-Fluorbenzaldehyd (149 mg, 1,2 mmol) und Dimethyl-(1-phenyl-4-trimethylsilanyloxymethylcyclohexyl)amin (306 mg, 1 mmol) in abs. DCM (20 ml) gelöst und auf 0 °C abgekühlt. Trimethylsilyltriflat (387 µl, 2 mmol) wurde bei 0 °C zugetropft und 60 min bei dieser Temperatur gerührt, bevor Triethylsilan (319 µl, 2 mmol) bei 0 °C zugetropft wurde. Unter langsamer Erwärmung auf RT wurde das Gemisch über Nacht gerührt. Zur Aufarbeitung wurde DCM (10 ml) zugegeben, mit 1 M NaOH (5 ml) versetzt und 15 min kräftig gerührt. Die organische Phase wurde abgetrennt, mit 1 M NaOH (2 x 2 ml) und mit Wasser (1 x 2 ml) gewaschen, getrocknet, filtriert und eingeengt. Der Rückstand wurde durch Chromatographie an Kieselgel gereinigt [40 g Kieselgel 60; Eluent: 600 ml EE/MeOH (1 : 1) und 600 ml MeOH/konz. $NH_3$ (400 : 1)]. Es wurden die beiden Diastereoisomere von [4-(4-Fluorbenzyloxymethyl)-1-phenylcyclohexyl]dimethylamin isoliert (17 mg unpolareres Diastereoisomer (gelbes Öl) und 106 mg polareres Diastereoisomer (gelbes Öl). 75 mg des polareren Diastereoisomers wurden in 2-Butanon (2 ml) unter Erwärmen gelöst, bei RT wurde Chlortrimethylsilan (83,4 µ) zugetropft und 2 h gerührt. Das Lösungsmittel wurde vollständig abdestilliert und der Rückstand mit trockenem Ether überschichtet und mechanisch von der Kolbenwand gelöst. Der so erhaltene Feststoff wurde abgesaugt, mit Ether (4 x 2 ml) gewaschen und getrocknet. Es wurden 79 mg des Hydrochlorids des polareren Diastereoisomers von [4-(4-Fluorbenzyloxymethyl)-1-phenylcyclohexyl]dimethylamin erhalten (grauweißer Feststoff, Smp. 176-181 °C).

**Untersuchungen zur Wirksamkeit der erfindungsgemäßen Verbindungen:**

Die in den folgenden Assays und Modellen erhobenen Daten sind in Tabelle 1 zusammengefaßt.

**Messung der ORL1-Bindung**

Die Cyclohexan-Derivate der allgemeinen Formel I wurde in einem Rezeptorbindungsassay mit $^3$H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von $^3$H-Nociceptin/Orphanin FQ betrug bei diesen Versuchen 0.5 nM. Die Bindungsassays wurden mit je 20 µg Membranprotein je 200 µl Ansatz in 50 mM Hepes, pH 7,4, 10 mM $MgCl_2$ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei RT und anschliessende Messung im Szintillationscounter Trilux (Wallac, Finnland), bestimmt. Die Affinität wird in Tabelle 1 als nanomolarer $K_i$-Wert in oder % Inhibition bei c=1 µM angegeben.

**Messung der μ-Bindung**

Die Rezeptoraffinität zum humanen μ-Opiatrezeptor wurde in einem homogenen Ansatz in Mikrotiterplatten bestimmt. Hierzu wurden Verdünnungsreihen des jeweils zu prüfenden substituierten spirocyclischen Cyclohexan-Derivates mit einer Rezeptormembranpräparation (15-40 μg Protein pro 250 μl Inkubationsansatz) von CHO-K1-Zellen, welche den humanen μ-Opiatrezeptor exprimieren (RB-HOM-Rezeptormembran-Präparation der Firma NEN, Zaventem, Belgien) in Gegenwart von 1 nmol/l des radioaktiven Liganden [$^3$H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 μl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 μmol/l Naloxon zugegeben. Nach Beendigung der neunzigminütigen Inkubationszeit wurden die Mikrotiterplatten für 20 Minuten bei 1000 g abzentrifugiert und die Radioaktivität in einem β-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen μ-Opiatrezeptor bei einer Konzentration der Prüfsubstanzen von 1 μmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I $IC_{50}$ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten.

**Analgesieprüfung im Tail-Flick-Test an der Maus**

Die Mäuse wurden jeweils einzeln in einen Testkäfig gesetzt und die Schwanzbasis dem fokussierten Wärmestrahl einer elektrischen Lampe (Tail-flick-Typ 50/08/1.bc, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Mäusen 3 bis 5 Sekunden betrug. Vor der Applikation der Lösungen enthaltend die erfindungsgemäße Verbindung bzw. der jeweiligen Vergleichslösungen wurden die Mäuse innerhalb von fünf Minuten zweimal vorgetestet und der Mittelwert dieser Messungen als Vortestmittelwert berechnet.

Die Lösungen der erfindungsgemäßen Verbindung der allgemeinen Formel I sowie die Vergleichslösungen wurden dann intravenös appliziert. Die Schmerzmessung wurde jeweils 10, 20, 40 und 60 Minuten nach der intravenösen Applikation durchgeführt. Die analgetische Wirkung wurde als Zunahme der Schmerzlatenz (% des maximal möglichen antinociceptiven Effektes) nach der folgenden Formel bestimmt:

$$[(T_1-T_0)/(T_2-T_0)] \times 100$$

Hierbei ist die Zeit $T_0$ die Latenzzeit vor der Applikation, die Zeit $T_1$ die Latenzzeit nach der Applikation der Wirkstoffkombination und die Zeit $T_2$ die maximale Expositionsdauer (12 Sekunden).

**Tabelle 1:**

| **Beispiel Nr.** | **ORL1** Ki [nM] oder % Hemmung [1 μM] | μ Ki [nM] oder % Hemmung [1 μM] | **Tail Flick** (Maus, i.v.) $ED_{50}$ [mg/kg] oder %Hemmung (Dosis [mg/kg]) |
|---|---|---|---|
| **1** | 26 | 4,7 | 87 |
| **2** | 370 | 78% | |
| **3** | 21 | 8,9 | |
| **4** | 250 | 40 | |

**Beispiel 5:**

**Parenterale Lösung eines erfindungsgemäßen 4-Hydroxymethyl-1-Aryl-Cyclohexylamin-Derivats**

1 g eines der erfindungsgemäßen 4-Hydroxymethyl-1-Aryl-Cyclohexylamin-Derivate, hier Beispielverbindung 1, wird in 1 l Wasser für Injektionszwecke bei Raumtemperatur gelöst und anschließend durch Zugabe von wasserfreier Glukose für Injektionszwecke auf isotone Bedingungen eingestellt.

## Patentansprüche

1. 4-Hydroxymethyl-1-Aryl-Cyclohexylamin-Derivate der allgemeinen Formel I,

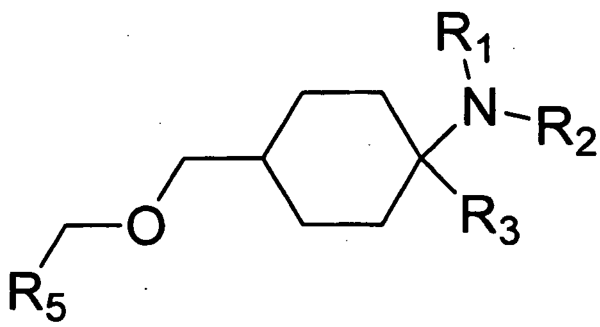

, worin

$R^1$ und $R^2$ unabhängig voneinander für H; $C_{1-8}$-Alkyl, gesättigt oder ungesättigt, verzweigt oder unverzweigt, einfach oder mehrfach substituiert oder unsubstituiert, stehen;
oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
wobei $R^6$ H; $C_{1-8}$-Alkyl, gesättigt oder ungesättigt, verzweigt oder unverzweigt, einfach oder mehrfach substituiert oder unsubstituiert, bedeutet,
$R^3$ für $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenen Aryl, $C_{3-8}$-Cycloalkyl oder Heteroaryl, jeweils unsubstituiert oder einfach oder mehrfach substituiert, steht;
$R^5$ für $C_{3-8}$-Cycloalkyl, Aryl oder Heteroaryl, jeweils unsubstituiert oder einfach oder mehrfach substituiert; $-CH_2R^{12}$, $-CH_2-CH_2R^{12}$, $-CH_2-CH_2-CH_2R^{12}$ steht
wobei $R^{12}$
$C_{3-8}$-Cycloalkyl, Aryl oder Heteroaryl, jeweils unsubstituiert oder einfach oder mehrfach substituiert, bedeutet,

und wobei im bezug auf Cycloakyl der Begriff auch gesättigte Cycloalkyle umfaßt, in denen ein oder zwei Kohlenstoffatome durch ein Heteroatom, S, N oder O ersetzt sind,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate.

2. 4-Hydroxymethyl-1-Aryl-Cyclohexylamin-Derivate gemäß Anspruch 1, **dadurch gekennzeichnet, dass**

$R^1$ und $R^2$ unabhängig voneinander für H; $C_{1-4}$-Alkyl, gesättigt oder ungesättigt, verzweigt oder unverzweigt, einfach oder mehrfach substituiert oder unsubstituiert, stehen; wobei $R^1$ und $R^2$ nicht beide H sein dürfen,
oder die Reste $R^1$ und $R^2$ zusammen einen Ring bilden und $(CH_2)_{4-5}$ bedeuten,
insbesondere

$R^1$ und $R^2$ unabhängig voneinander für Methyl oder Ethyl stehen oder die Reste $R^1$ und $R^2$ zusammen einen Ring bilden und $(CH_2)_5$ bedeuten.

3. 4-Hydroxymethyl-1-Aryl-Cyclohexylamin-Derivate gemäß Anspruch 1, **dadurch gekennzeichnet, dass** $R^1$ und $R^2$ unabhängig voneinander für $CH_3$ oder H stehen, wobei $R^1$ und $R^2$ nicht gleichzeitig H bedeuten.

4. 4-Hydroxymethyl-1-Aryl-Cyclohexylamin-Derivate gemäß einem der Ansprüche 1-3, **dadurch gekennzeichnet, dass**

$R^3$ für $C_{3-8}$-Cycloalkyl, Aryl oder Heteroaryl, jeweils unsubstituiert oder einfach oder mehrfach substituiert; über eine gesättigte oder ungesättigte, unverzweigte, substituierte oder unsubstituierte $C_{1-2}$-Alkyl-Gruppe gebundenen Aryl, $C_{3-8}$-Cycloalkyl oder Heteroaryl, jeweils unsubstituiert oder einfach oder mehrfach substituiert, steht;
vorzugsweise
$R^3$ Cyclopentyl, Cyclohexyl, Phenyl, Benzyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyridyl, Pyrimidyl oder Pyrazinyl, jeweils unsubstituiert oder einfach oder mehrfach substituiert; über eine gesättigte, unverzweigte $C_{1-2}$-Alkyl-Gruppe gebundenen $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert, bedeutet;
insbesondere
$R^3$ Phenyl, Furyl, Thiophenyl, Naphthyl, Benzyl, Benzofuranyl, Indolyl, Indanyl, Benzodioxanyl, Benzodioxolanyl, Pyridyl, Pyrimidyl, Pyrazinyl oder Benzothiophenyl, jeweils unsubstituiert oder einfach oder mehrfach substituiert; über eine gesättigte, unverzweigte $C_{1-2}$-Alkyl-Gruppe gebundenen Phenyl, Furyl oder Thiophenyl, jeweils unsubstituiert oder einfach oder mehrfach substituiert, bedeutet.

5. 4-Hydroxymethyl-1-Aryl-Cyclohexylamin-Derivate gemäß einem der Ansprüche 1 bis 3, **dadurch gekennzeichnet, dass** $R^3$ für Phenyl, Thiophenyl, Pyridyl oder Benzyl, jeweils substituiert oder unsubstituiert, steht, besonders bevorzugt für Phenyl.

6. 4-Hydroxymethyl-1-Aryl-Cyclohexylamin-Derivate gemäß einem der Ansprüche 1 bis 5, **dadurch gekennzeichnet, dass**

$R^5$ für $C_{3-8}$-Cycloalkyl, Aryl oder Heteroaryl, jeweils unsubstituiert oder einfach oder mehrfach substituiert, steht;
vorzugsweise
$R^5$ Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder
Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert, bedeutet;
insbesondere
$R^5$ Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert, bedeutet.

7. 4-Hydroxymethyl-1-Aryl-Cyclohexylamin-Derivate gemäß einem der Ansprüche 1 bis 5, **dadurch gekennzeichnet, dass**

$R^5$ für $-CH_2R^{12}$, $-CH_2-CH_2R^{12}$, $-CH_2-CH_2-CH_2R^{12}$ steht,
vorzugsweise
$R^5$ $-CH_2R^{12}$, $-CH_2-CH_2R^{12}$ bedeutet,
insbesondere
$R^5$ $-CH_2R^{12}$ bedeutet, wobei
$R^{12}$ für $C_{3-8}$-Cycloalkyl, Aryl oder Heteroaryl, jeweils unsubstituiert oder einfach oder mehrfach substituiert, steht.

8. 4-Hydroxymethyl-1-Aryl-Cyclohexylamin-Derivate gemäß Anspruch 7, **dadurch gekennzeichnet, dass**

$R^{12}$ Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert, bedeutet;
insbesondere
$R^{12}$ Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert, bedeutet.

**9.** 4-Hydroxymethyl-1-Aryl-Cyclohexylamin-Derivate gemäß einem der Ansprüche 1-8 aus der Gruppe

(4-Benzyloxymethyl-1-phenylcyclohexyl)dimethylamin Hydrochlorid, unpolareres Diastereoisomer
(4-Benzyloxymethyl-1-phenylcyclohexyl)dimethylamin Hydrochlorid, polareres Diastereoisomer
[4-(4-Fluorbenzyloxymethyl)-1-phenylcyclohexyl]dimethylamin Hydrochlorid, unpolareres Diastereoisomer
[4-(4-Fluorbenzyloxymethyl)-1-phenylcyclohexyl]dimethylamin Hydrochlorid, polareres Diastereoisomer,
gegebenenfalls auch in Form ihrer Mischungen.

**10.** Arzneimittel enthaltend mindestens ein 4-Hydroxymethyl-1-Aryl-Cyclohexylamin-Derivat gemäß einem der Ansprüche 1 bis 9 sowie gegebenenfalls geeignete Zusatz- und/oder Hilfsstoffe und/oder gegebenenfalls weitere Wirkstoffe.

**11.** Verwendung eines 4-Hydroxymethyl-1-Aryl-Cyclohexylamin-Derivats gemäß einem der Ansprüche 1 bis 9 zur Herstellung eines Arzneimittels zur Behandlung von Schmerz, insbesondere von akutem, viszeralem, neuropathischem oder chronischem Schmerz.

**12.** Verwendung eines 4-Hydroxymethyl-1-Aryl-Cyclohexylamin-Derivats gemäß einem der Ansprüche 1 bis 9 zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

**13.** Verfahren zur Herstellung von 4-Hydroxymethyl-1-Aryl-Cyclohexylamin-Derivaten gemäß einem der Ansprüche 1 bis 9, **dadurch gekennzeichnet, dass** 4-Aminocyclohexancarbaldehyd-Derivate mit einem Reduktionsmittel, beispielsweise Natriumborhydrid, reduziert werden und darauf folgend mit einem Alkylierungsmittel $R^5CH_2X$ in Gegenwart von Base, beispielsweise Kalium-tert-butanolat, umgesetzt werden.

**14.** Verfahren zur Herstellung von 4-Hydroxymethyl-1-Aryl-Cyclohexylamin-Derivaten gemäß einem der Ansprüche 1 bis 9, **dadurch gekennzeichnet, dass** 4-Aminocyclohexancarbaldehyd-Derivate unter Zugabe eines Reduktionsmittels, beispielsweise Natriumborhydrid, zu den entsprechenden Alkoholen reduziert, und die Alkohole darauf folgend mit Trimethylchlorsilan und Hexamethyldisilazan sowie anschließend einem Aldehyd $R^5CHO$ unter Zugabe von Trimethylsilyltriflat und Triethylsilan reduktiv verethert werden.

**15.** Verfahren zur Herstellung von 4-Aminocyclohexancarbaldehyd-Derivaten gemäß einem der Ansprüche 13 oder 14, **dadurch gekennzeichnet, dass** 4-Aminocyclohexanon-Derivate mit Methoxymethyltriphenylphosphoniumchlorid und einer Base, beispielsweise Natriumhydrid, und anschließend mit wäßriger Säure, beispielsweise HCl, umgesetzt wird.

## Claims

**1.** 4-Hydroxymethyl-1-arylcyclohexylamine derivatives of general formula I:

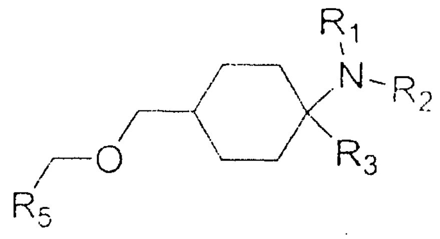

in which

$R^1$ and $R^2$ independently of one another are H; or $C_{1-8}$-alkyl which is saturated or unsaturated, branched or unbranched and monosubstituted, polysubstituted or unsubstituted,
or the radicals $R^1$ and $R^2$ together form a ring and are $CH_2CH_2OCH_2CH_2$, $CH_2CH_2NR^6CH_2CH_2$ or $(CH_2)_{3-6}$, $R^6$ being H; or $C_{1-8}$-alkyl which is saturated or unsaturated, branched or unbranched and monosubstituted, polysubstituted or unsubstituted;
$R^3$ is $C_{1-8}$-alkyl or $C_{3-8}$-cycloalkyl, each of which is saturated or unsaturated, branched or unbranched and monosubstituted, polysubstituted or unsubstituted; aryl or heteroaryl, each of which is unsubstituted, monosubstituted or polysubstituted; or aryl, $C_{3-8}$-cycloalkyl or heteroaryl bonded via a saturated or unsaturated, branched or unbranched and substituted or unsubstituted $C_{1-4}$-alkyl group, each of which is unsubstituted, monosubstituted or polysubstituted; and $R^5$ is $C_{3-8}$-cycloalkyl, aryl or heteroaryl, each of which is unsubstituted, monosubstituted or polysubstituted; or $-CH_2R^{12}$, $-CH_2-CH_2R^{12}$ or $-CH_2-CH_2-CH_2R^{12}$,
$R^{12}$ being $C_{3-8}$-cycloalkyl, aryl or heteroaryl, each of which is unsubstituted, monosubstituted or polysubstituted, and the term "cycloalkyl" also including saturated cycloalkyls in which one or two carbon atoms have been replaced by a heteroatom S, N or O,

optionally in the form of their racemates or pure stereoisomers, especially enantiomers or diastereoisomers, or in the form of mixtures of the stereoisomers, especially enantiomers or diastereoisomers, in any desired mixing ratio; and in the form of their acids or bases or in the form of their salts, especially physiologically acceptable salts or salts of physiologically acceptable acids or cations, or in the form of their solvates, especially hydrates.

**2.** 4-Hydroxymethyl-1-arylcyclohexylamine derivatives according to Claim 1, **characterized in that**

$R^1$ and $R^2$ independently of one another are H; or $C_{1-4}$-alkyl which is saturated or unsaturated, branched or unbranched and monosubstituted, polysubstituted or unsubstituted, it being impossible for both $R^1$ and $R^2$ to be H,
or the radicals $R^1$ and $R^2$ together form a ring and are $(CH_2)_{4-5}$,
and especially
$R^1$ and $R^2$ independently of one another are methyl or ethyl, or the radicals $R^1$ and $R^2$ together form a ring and are $(CH_2)_5$.

**3.** 4-Hydroxymethyl-1-arylcyclohexylamine derivatives according to Claim 1, **characterized in that** $R^1$ and $R^2$ independently of one another are $CH_3$ or H, $R^1$ and $R^2$ not being H simultaneously.

**4.** 4-Hydroxymethyl-1-arylcyclohexylamine derivatives according to one of Claims 1-3, **characterized in that**

$R^3$ is $C_{3-8}$-cycloalkyl, aryl or heteroaryl, each of which is unsubstituted, monosubstituted or polysubstituted; or aryl, $C_{3-8}$-cycloalkyl or heteroaryl bonded via a saturated or unsaturated, unbranched and substituted or unsubstituted $C_{1-2}$-alkyl group, each of which is unsubstituted, monosubstituted or polysubstituted,
preferably
$R^3$ is cyclopentyl, cyclohexyl, phenyl, benzyl, naphthyl, anthracenyl, thiophenyl, benzothiophenyl, furyl, benzofuranyl, benzodioxolanyl, indolyl, indanyl, benzodioxanyl, pyrrolyl, pyridyl, pyrimidyl or pyrazinyl, each of which is unsubstituted, monosubstituted or polysubstituted; or a $C_{5-6}$-cycloalkyl, phenyl, naphthyl, anthracenyl, thiophenyl, benzothiophenyl, pyridyl, furyl, benzofuranyl, benzodioxolanyl, indolyl, indanyl, benzodioxanyl, pyrrolyl, pyrimidyl or pyrazinyl bonded via a saturated unbranched $C_{1-2}$-alkyl group, each of which is unsubstituted, monosubstituted or polysubstituted,
and especially
$R^3$ is phenyl, furyl, thiophenyl, naphthyl, benzyl, benzofuranyl, indolyl, indanyl, benzodioxanyl, benzodioxolanyl, pyridyl, pyrimidyl, pyrazinyl or benzothiophenyl, each of which is unsubstituted, monosubstituted or polysubstituted; or phenyl, furyl or thiophenyl bonded via a saturated unbranched $C_{1-2}$-alkyl group, each of which is unsubstituted, monosubstituted or polysubstituted.

**5.** 4-Hydroxymethyl-1-arylcyclohexylamine derivatives according to one of Claims 1 to 3, **characterized in that** $R^3$ is phenyl, thiophenyl, pyridyl or benzyl, each of which is substituted or unsubstituted, phenyl being particularly preferred.

**6.** 4-Hydroxymethyl-1-arylcyclohexylamine derivatives according to one of Claims 1 to 5, **characterized in that**

$R^5$ is $C_{3-8}$-cycloalkyl, aryl or heteroaryl, each of which is unsubstituted, monosubstituted or polysubstituted,
preferably
$R^5$ is cyclobutyl, cyclopropyl, cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl, pyrimidyl, fluorenyl,
fluoranthenyl, benzothiazolyl, benzotriazolyl, benzo[1,2,5]thiazolyl, 1,2-dihydroacenaphthenyl, pyridinyl, furanyl, benzofuranyl, pyrazolinonyl, oxopyrazolinonyl, dioxolanyl, adamantyl, pyrimidinyl, quinolinyl, isoquinolinyl, phthalazinyl or quinazolinyl, each of which is unsubstituted, monosubstituted or polysubstituted,
and especially
$R^5$ is cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, each of which is unsubstituted, monosubstituted or polysubstituted.

**7.** 4-Hydroxymethyl-1-arylcyclohexylamine derivatives according to one of Claims 1 to 5, **characterized in that**

$R^5$ is $-CH_2R^{12}$, $-CH_2-CH_2R^{12}$ or $-CH_2-CH_2-CH_2R^{12}$,
preferably
$R^5$ is $-CH_2R^{12}$ or $-CH_2-CH_2R^{12}$,
and especially
$R^5$ is $-CH_2R^{12}$,
$R^{12}$ being $C_{3-8}$-cycloalkyl, aryl or heteroaryl, each of which is unsubstituted, monosubstituted or polysubstituted.

**8.** 4-Hydroxymethyl-1-arylcyclohexylamine derivatives according to Claim 7, **characterized in that**

$R^{12}$ is cyclobutyl, cyclopropyl, cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl, pyrimidyl, fluorenyl, fluoranthenyl, benzothiazolyl, benzotriazolyl, benzo[1,2,5]thiazolyl, 1,2-dihydroacenaphthenyl, pyridinyl, furanyl, benzofuranyl, pyrazolinonyl, oxopyrazolinonyl, dioxolanyl, adamantyl, pyrimidinyl, quinolinyl, isoquinolinyl, phthalazinyl or quinazolinyl, each of which is unsubstituted, monosubstituted or polysubstituted,
and especially
$R^{12}$ is cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, each of which is unsubstituted, monosubstituted or polysubstituted.

**9.** 4-Hydroxymethyl-1-arylcyclohexylamine derivatives according to one of Claims 1-8 from the group comprising

(4-benzyloxymethyl-1-phenylcyclohexyl)dimethylamine hydrochloride, less polar diastereoisomer,
(4-benzyloxymethyl-1-phenylcyclohexyl)dimethylamine hydrochloride, more polar diastereoisomer,
[4-(4-fluorobenzyloxymethyl)-1-phenylcyclohexyl]-dimethylamine hydrochloride, less polar diastereoisomer, and
[4-(4-fluorobenzyloxymethyl)-1-phenylcyclohexyl]-dimethylamine hydrochloride, more polar diastereoisomer,

optionally also in the form of their mixtures.

10. Drug containing at least one 4-hydroxymethyl-1-arylcyclohexylamine derivative according to one of Claims 1 to 9, optionally suitable additives and/or auxiliary substances and/or optionally other active substances.

11. Use of a 4-hydroxymethyl-1-arylcyclohexylamine derivative according to one of Claims 1 to 9 to prepare a drug for the treatment of pain, especially acute, visceral, neuropathic or chronic pain.

12. Use of a 4-hydroxymethyl-1-arylcyclohexylamine derivative according to one of Claims 1 to 9 to prepare a drug for the treatment of anxiety states, stress and stress-related syndromes, depression, epilepsy, Alzheimer's disease, senile dementia, general cognitive dysfunctions, learning and memory disorders (as a nootropic), withdrawal symptoms, alcohol and/or narcotic and/or drug abuse and/or dependence, sexual dysfunctions, cardiovascular diseases, hypotension, hypertension, tinnitus, pruritus, migraine, hearing difficulty, deficient intestinal motility, disturbed ingestion, anorexia, obesity, locomotor disturbances, diarrhoea, cachexia and urinary incontinence, or as a muscle relaxant, anticonvulsive or anaesthetic, or for coadministration in the case of treatment with an opioid analgesic or an anaesthetic, for diuresis or antinatriuresis, and anxiolysis, for the modulation of motor activity, for the modulation of neurotransmitter release and the treatment of associated neurodegenerative diseases, for the treatment of withdrawal symptoms and/or for reduction of the addiction potential of opioids.

13. Process for the preparation of 4-hydroxymethyl-1-arylcyclohexylamine derivatives according to one of Claims 1 to 9, **characterized in that** 4-amino-cyclohexanecarbaldehyde derivatives are reduced with a reducing agent, for example sodium borohydride, and then reacted with an alkylating agent $R^5CH_2X$ in the presence of a base, for example potassium tert-butylate.

14. Process for the preparation of 4-hydroxymethyl-1-arylcyclohexylamine derivatives according to one of Claims 1 to 9, **characterized in that** 4-amino-cyclohexanecarbaldehyde derivatives are reduced by adding a reducing agent, for example sodium borohydride, to give the corresponding alcohols, and the alcohols are subsequently reductively etherified with trimethylchlorosilane, hexamethyldisilazane and then an aldehyde $R^5CHO$, with the addition of trimethylsilyl triflate and triethylsilane.

15. Process for the preparation of 4-aminocyclohexane-carbaldehyde derivatives according to Claim 13 or 14, **characterized in that** 4-aminocyclohexanone derivatives are reacted with methoxymethyltriphenylphosphonium chloride and a base, for example sodium hydride, and then with an aqueous acid, for example HCl.

## Revendications

1. Dérivés de 4-hydroxyméthyl-1-aryl-cyclohexylamines de formule générale I

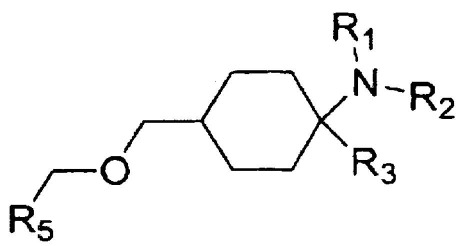

dans laquelle

$R^1$ et $R^2$ représentent, indépendamment l'un de l'autre, H ; un reste alkyle en $C_1$ à $C_8$, saturé ou non saturé, ramifié ou non ramifié, substitué une ou plusieurs fois ou non substitué ;
ou bien les restes $R^1$ et $R^2$ forment ensemble un noyau et représentent $CH_2CH_2OCH_2CH_2$, $CH_2CH_2NR^6CH_2CH_2$ ou $(CH_2)_{3-6}$,
où $R^6$ représente H ; un reste alkyle en $C_1$ à $C_8$, saturé ou non saturé, ramifié ou non ramifié, substitué une ou plusieurs fois ou non substitué,
$R^3$ représente un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste aryle, cycloalkyle en $C_3$ à $C_8$ ou hétéroaryle lié par l'intermédiaire

d'un groupe alkyle en $C_1$ à $C_4$ saturé ou non saturé, ramifié ou non ramifié, substitué ou non substitué, chaque reste étant non substitué ou substitué une ou plusieurs fois ;
$R^5$ représente un reste cycloalkyle en $C_3$ à $C_8$, aryle ou hétéroaryle, chacun non substitué ou substitué une ou plusieurs fois ; un groupe $-CH_2R^{12}$, $-CH_2-CH_2R^{12}$, $-CH_2-CH_2-CH_2R^{12}$
où $R^{12}$ représente un reste cycloalkyle en $C^3$ à $C^8$, aryle ou hétéroaryle, chacun non substitué ou substitué une ou plusieurs fois,
et à propos d'un reste cycloalkyle, ce terme comprend aussi des restes cycloalkyle saturés dans lesquels un ou deux atomes de carbone sont remplacés par un hétéroatome S, N ou O,
éventuellement sous forme de leurs racémates, de leurs stéréoisomères purs, particulièrement des énantiomères ou des diastéréoisomères, ou sous forme de mélanges des stéréoisomères, en particulier des énantiomères ou des diastéréoisomères, dans un rapport de mélange quelconque ; sous forme de leurs acides ou de leurs bases ou sous forme de leurs sels, en particulier des sels physiologiquement acceptables ou de sels d'acides ou de cations physiologiquement acceptables ; ou sous forme de leurs produits de solvatation, en particulier des hydrates.

**2.** Dérivés de 4-hydroxyméthyl-1-aryl-cyclohexylamines suivant la revendication 1, **caractérisés en ce que**

$R^1$ et $R^2$ représentent indépendamment l'un de l'autre H ; un reste alkyle en $C_1$ à $C_4$, saturé ou non saturé, ramifié ou non ramifié, substitué une ou plusieurs fois ou non substitué ; $R^1$ et $R^2$ ne pouvant pas représenter tous deux H,
ou bien les restes $R^1$ et $R^2$ forment ensemble un noyau et représentent un groupe $(CH_2)_{4-5}$,
en particulier
$R^1$ et $R^2$ représentent indépendamment l'un de l'autre un reste méthyle ou éthyle ou bien les restes $R^1$ et $R^2$ forment ensemble un noyau et représentent un groupe $(CH_2)_5$.

**3.** Dérivés de 4-hydroxyméthyl-1-aryl-cyclohexylamines suivant la revendication 1, **caractérisés en ce que** $R^1$ et $R^2$ représentent, indépendamment l'un de l'autre, $CH_3$ ou H, $R^1$ et $R^2$ ne pouvant pas représenter en même temps H.

**4.** Dérivés de 4-hydroxyméthyl-1-aryl-cyclohexylamines suivant l'une des revendications 1 à 3, **caractérisés en ce que**

$R^3$ représente un reste cycloalkyle en $C_3$ à $C_8$, aryle ou hétéroaryle, chacun non substitué ou substitué une ou plusieurs fois ; un reste aryle, cycloalkyle en $C_3$ à $C_8$ ou hétéroaryle lié par l'intermédiaire d'un groupe alkyle en $C_1$ ou $C_2$ saturé ou non saturé, non ramifié, substitué ou non substitué, chaque reste étant non substitué ou substitué une ou plusieurs fois ;
avantageusement
$R^3$ représente un reste cyclopentyle, cyclohexyle, phényle, benzyle, naphtyle, anthracényle, thiophényle, benzothiophényle, furyle, benzofurannyle, benzodioxolannyle, indolyle, indanyle, benzodioxannyle, pyrrolyle, pyridyle, pyrimidyle ou pyrazinyle, chacun non substitué ou substitué une ou plusieurs fois ; ou un reste cycloalkyle en $C_5$ ou $C_6$, phényle, naphtyle, anthracényle, thiophényle, benzothiophényle, pyridyle, furyle, benzofurannyle, benzodioxolannyle, indolyle, indanyle, benzodioxannyle, pyrrolyle, pyrimidyle ou pyrazinyle lié par l'intermédiaire d'un groupe alkyle en $C_1$ ou $C_2$ saturé non ramifié, chaque reste étant non substitué ou substitué une ou plusieurs fois ;
en particulier,
$R^3$ représente un reste phényle, furyle, thiophényle, naphtyle, benzyle, benzofurannyle, indolyle indanyle, benzodioxannyle, benzodioxolannyle, pyridyle, pyrimidyle ou pyrazinyle ou benzothiophényle, chacun non substitué ou substitué une ou plusieurs fois ; un reste phényle, furyle, ou thiophényle lié par l'intermédiaire d'un groupe alkyle en $C_1$ ou $C_2$ saturé non ramifié, chaque reste étant non substitué ou substitué une ou plusieurs fois.

**5.** Dérivés de 4-hydroxyméthyl-1-aryl-cyclohexylamines suivant l'une des revendications 1 à 3, **caractérisés en ce que** R3 représente un reste phényle, thiophényle, pyridyle ou benzyle, chacun substitué ou non substitué, très avantageusement un reste phényle.

**6.** Dérivés de 4-hydroxyméthyl-1-aryl-cyclohexylamines suivant l'une des revendications 1 à 5, **caractérisés en ce que**

$R^5$ représente un reste cycloalkyle en $C_3$ à $C_8$, aryle ou hétéroaryle, chacun non substitué ou substitué une ou plusieurs fois ;
avantageusement
$R^5$ représente un reste cyclobutyle, cyclopropyle, cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]thiazolyle ou 1,2-dihydroacénaphtényle, pyridinyle, furannyle, benzofurannyle, pyrazolinonyle, oxopyrazolinonyle, dioxolannyle, adamantyle, pyrimidinyle, quinolinyle, isoquinolinyle, phtalazinyle ou quinazolinyle, chacun non substitué ou substitué une ou plusieurs fois ;

en particulier

$R^5$ représente un reste cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, chacun non substitué ou substitué une ou plusieurs fois.

**7.** Dérivés de 4-hydroxyméthyl-1-aryl-cyclohexylamines suivant l'une des revendications 1 à 5, **caractérisés en ce que**

$R^5$ représente $-CH_2R^{12}$, $-CH_2-CH_2R^{12}$, $-CH_2-CH_2-CH_2R^{12}$, avantageusement

$R^5$ représente $-CH_2R^{12}$, $-CH_2-CH_2R^{12}$,

en particulier

$R^5$ représente $-CH_2R^{12}$, où

$R^{12}$ est un reste cycloalkyle en $C_3$ à $C_8$, aryle ou hétéroaryle, chacun non substitué ou substitué une ou plusieurs fois.

**8.** Dérivés de 4-hydroxyméthyl-1-aryl-cyclohexylamines suivant la revendication 7, **caractérisés en ce que**

$R^{12}$ représente un reste cyclobutyle, cyclopropyle, cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]thiazolyle ou 1,2-dihydroacénaphtényle, pyridinyle, furannyle, benzofurannyle, pyrazolinonyle, oxopyrazolinonyle, dioxolannyle, adamantyle, pyrimidinyle, quinolinyle, isoquinolinyle, phtalazinyle ou quinazolinyle, chacun non substitué ou substitué une ou plusieurs fois,

en particulier

$R^{12}$ représente un reste cyclopentyle, cyclohexyle, cycloheptyle, cyclooctyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, chacun non substitué ou substitué une ou plusieurs fois.

**9.** Dérivés de 4-hydroxyméthyl-1-aryl-cyclohexylamines suivant l'une des revendications 1 à 8, du groupe

chlorhydrate de (4-benzyloxyméthyl-1-phénylcyclohexyl)-diméthylamine, diastéréoisomère le moins polaire ;

chlorhydrate de (4-benzyloxyméthyl-1-phénylcyclohexyl)-diméthylamine, diastéréoisomère le plus polaire ;

chlorhydrate de [4-(4-fluorobenzyloxyméthyl)-1-phénylcyclohexyl]diméthylamine, diastéréoisomère le moins polaire ;

chlorhydrate de [4-(4-fluorobenzyloxyméthyl)-1-phénylcyclohexyl]diméthylamine, diastéréoisomère le plus polaire,

le cas échéant également sous forme de leurs mélanges.

**10.** Médicament contenant au moins un dérivé de 4-hydroxyméthyl-1-aryl-cyclohexylamine suivant l'une des revendications 1 à 9, ainsi que, le cas échéant, des additifs et/ou des substances auxiliaires convenables et/ou éventuellement d'autres substances actives.

**11.** Utilisation d'un dérivé de 4-hydroxyméthyl-1-aryl-cyclohexylamine suivant l'une des revendications 1 à 9 pour la préparation d'un médicament destiné au traitement de la douleur, en particulier d'une douleur aiguë, viscérale, neuropathique ou chronique.

**12.** Utilisation d'un dérivé de 4-hydroxyméthyl-1-aryl-cyclohexylamine suivant l'une des revendications 1 à 9 pour la préparation d'un médicament destiné au traitement d'états d'angoisse, de stress et de syndromes liés au stress, de dépressions, de l'épilepsie, de la maladie d'Alzheimer, de la démence sénile, de dysfonctions cognitives générales, de troubles de l'apprentissage et de la mémoire (comme agent nootrope), de manifestations de privation, d'abus et/ou de dépendance vis-à-vis de l'alcool et/ou des drogues et/ou des médicaments, de dysfonctions sexuelles, de maladies cardiovasculaires, d'hypotension, d'hypertension, d'acouphènes, de prurit, de migraines, de surdité, d'insuffisance de la motilité intestinale, de troubles de l'absorption de nourriture, de l'anorexie, de l'obésité, de troubles locomoteurs, de la diarrhée, de la cachexie, de l'incontinence urinaire et comme myorelaxant, anticonvulsif et anesthésique ou en vue d'une coadministration lors d'un traitement avec un analgésique opioïde et avec un anesthésique, en vue de la diurèse et de l'antinatriurèse, de l'anxiolyse, en vue de la modulation de l'activité motrice, en vue de la modulation de la sécrétion des neuromédiateurs et du traitement de maladies neurodégénératives qui y sont liées, en vue du traitement de manifestations de privation et/ou en vue de réduire le potentiel de dépendance envers les opiacés.

**13.** Procédé de préparation de dérivés de 4-hydroxyméthyl-1-aryl-cyclohexylamines suivant l'une des revendications 1 à 9, **caractérisé en ce qu'**on réduit des dérivés du 4-aminocyclohexanecarbaldéhyde avec un agent réducteur, par exemple le borohydrure de sodium, après quoi on les fait réagir avec un agent d'alkylation $R^5CH_2X$ en présence d'une base, par exemple le tertiobutanolate de potassium.

**14.** Procédé de préparation de dérivés de 4-hydroxyméthyl-1-aryl-cyclohexylamines suivant l'une des revendications 1 à 9, **caractérisé en ce qu'**on réduit des dérivés du 4-aminocyclohexanecarbaldéhyde avec addition d'un agent réducteur, par exemple le borohydrure de sodium, en les alcools correspondants, puis on éthérifie les alcools par voie de réduction avec le triméthylchlorosilane et l'hexaméthyldisilasane, et ensuite avec un aldéhyde $R^5CHO$ avec addition de triflate de triméthylsilyle et de triéthylsilane.

**15.** Procédé de préparation de dérivés du 4-aminocyclohexanecarbaldéhyde suivant l'une des revendications 13 ou 14, **caractérisé en ce qu'**on fait réagir des dérivés de 4-aminocyclohexanone avec le chlorure de méthoxyméthyltriphénylphosphonium et une base, par exemple l'hydrure de sodium, et ensuite avec un acide, par exemple HCl, en solution aqueuse.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9936421 A
- WO 0290317 A


### In der Beschreibung aufgeführte Nicht-Patentliteratur


- **Meunier et al.** *Nature,* 1995, vol. 377, 532-535
- **Reinscheid et al.** *Science,* 1995, vol. 270, 792-794
- **Mogil et al.** *Neuroscience,* 1996, vol. 75, 333-337
- **Jenck et al.** *Proc. Natl. Acad. Sci. USA,* 1997, vol. 94, 14854-14858
- **King et al.** *Neurosci. Lett.,* 1997, vol. 223, 113-116
- **Abdulla ; Smith.** *J. Neurosci.,* 1998, vol. 18, 9685-9694
- **Manabe et al.** *Nature,* 1997, vol. 394, 577-581
- **Nishi et al.** *EMBO J.,* 1997, vol. 16, 1858-1864
- **Calo et al.** *Br.J. Pharmacol.,* 2000, vol. 129, 1261-1283
- **Lednicer et al.** *J. Med. Chem.,* 1980, vol. 23, 424-430
- **Ardati et al.** *Mol. Pharmacol.,* 1997, vol. 51, 816-824